# EUROPÄISCHE PATENTSCHRIFT

(11) **EP 3 752 820 B1**
(45) Veröffentlichungstag und Bekanntmachung des Hinweises auf die Patenterteilung: **27.05.2026**
(21) Anmeldenummer: 19708939.4
(22) Anmeldetag: 14.02.2019
(51) Int. Cl.: G01N 23/04, G01N 33/36, D06F 93/00, G01V 5/00, D06F 39/00, G01N 21/3581

(54) **VERFAHREN ZUM INSPIZIEREN VON TEXTILEN GEGENSTÄNDEN**
METHOD FOR INSPECTING TEXTILE ARTICLES
PROCÉDÉ D'INSPECTION D'ARTICLES TEXTILES

(30) Priorität: 16.02.2018 DE 102018001192; 14.08.2018 DE 102018006395; 11.09.2018 DE 102018007184
(43) Veröffentlichungstag der Anmeldung: 23.12.2020
(73) Patentinhaber: Herbert Kannegiesser GmbH, 32602 Vlotho (DE)
(72) Erfinder: BRINGEWATT, Wilhelm, 32457 Porta Westfalica (DE); HEINZ, Engelbert, 32602 Vlotho (DE)
(74) Vertreter: Hoener, Matthias
(86) Internationale Anmeldenummer: PCT/EP2019/000042
(87) Internationale Veröffentlichungsnummer: WO 2019/158267

(56) Entgegenhaltungen:
- EP-A1- 3 235 944
- CN-A- 107 545 569
- CN-A- 107 653 625
- US-A1- 2019 065 915

## Beschreibung

Die Erfindung betrifft ein Verfahren zum Inspizieren von textilen Gegenständen gemäß dem Oberbegriff des Anspruchs 1.

Textile Gegenstände, insbesondere benutzte textile Gegenstände, wie Schmutzwäsche, beispielsweise Bekleidungsstücke, Berufsbekleidungsstücke, Flachwäsche oder dergleichen, müssen vor dem Reinigen, insbesondere Waschen, auf Fremdkörper inspiziert werden. Anschließend müssen Fremdkörper aufweisende textile Gegenstände von den Fremdkörpern befreit werden.

Das Inspizieren der textilen Gegenstände auf Fremdkörper und die Entfernung derselben erfolgen bislang manuell. Das ist kostenintensiv und benötigt relativ viel Zeit, wodurch die Taktdauer insbesondere des Waschvorgangs sich unnötig in die Länge zieht.

Aus der EP 3 235 944 A1 ist es bereits bekannt, textile Gegenstände insbesondere auf das Vorhandensein eventueller Fremdkörper automatisch zu inspizieren. Dazu werden die textilen Gegenstände mit Terahertz-Strahlung beaufschlagt. Die reflektierte bzw. transmittierte Strahlung wird energieaufgelöst detektiert und dabei ein Absorptionsspektrum vom jeweiligen textilen Gegenstand erstellt. Anhand der dabei detektierten Absorptionslinien werden zum Beispiel Fremdgegenstände in den Wäschestücken identifiziert.

Aus der CN 107 545 569 A ist bereits bekannt, Röntgenstrahlen zu verwenden, um Fremdkörper verschiedener Art in Textilgegenständen zu identifizieren.

Der Erfindung liegt die Aufgabe zugrunde, ein Verfahren zum Inspizieren von textilen Gegenständen, insbesondere benutzten textilen Gegenständen, zu schaffen, das ein zuverlässiges Inspizieren der textilen Gegenstände auf das Vorhandensein von Fremdkörpern automatisch zeitsparend ermöglicht.

Ein Verfahren zur Lösung der Aufgabe weist die Maßnahmen des Anspruchs 1 auf. Bei diesem Verfahren ist es vorgesehen, dass die Inspektion eines jeweiligen textilen Gegenstands durch elektromagnetische Strahlen unterschiedlicher Spektren unterschiedlicher Energie bzw. Intensität erfolgt. Das lässt eine quantitative Röntgenbildgebung von den durchstrahlten textilen Gegenständen zu. Dadurch ist eine zuverlässige Detektion von Fremdkörpern gewährleistet. Insbesondere sind unterschiedliche nicht-textile Materialien ermittelbar und/oder abbildbar.

Weiterhin ist es vorgesehen, dass zu jedem unterschiedlichen Spektrum, womit der gleiche textile Gegenstand inspiziert wird, eine eigene Aufnahme, insbesondere ein Bild des durchleuchteten textilen Gegenstands, erzeugt wird. Durch einen Vergleich, insbesondere einen automatischen, rechnergestützten Vergleich, erfolgt quasi eine "Verrechnung" der unterschiedlichen Aufnahmen bzw. Bilddaten, wodurch mit großer Genauigkeit Fremdkörper erkennbar werden. Es können so zuverlässiger und vorzugsweise vollständig Fremdkörper unterschiedlichster Art und unterschiedlicher Materialien wie beispielsweise metallischen Materialien einerseits und nichtmetallischem Materialien andererseits, ermittelt werden. Vor allem ist es so möglich, Fremdkörper zu erkennen, die bei einer Inspektion mit nur einem Spektrum von Röntgenstrahlen nicht feststellbar wären. Alternativ ist es denkbar, die Aufnahmen jedes Spektrums des gleichen textilen Gegenstands zu überlagern und dadurch ein gemeinsames Bild, also quasi ein Gesamtbild, zu erzeugen.

Bevorzugt ist es vorgesehen, die unterschiedlichen Röntgenspektren gleichzeitig zu erzeugen und durch separate Detektoren oder nur einen Detektor mit mehreren Detektorbereichen für jeweils ein Spektrum bildlich aufzunehmen und/oder visuell darzustellen. Hierbei werden immer alle textilen Gegenstände und Fremdkörper von allen Spektren durchstrahlt. Bei dieser Vorgehensweise brauchen vom Strahlungsgenerator keine verschiedenen Spektren erzeugt zu werden. Es reicht im einfachsten Falle eine Strahlenquelle mit nur einem einzigen Spektrum. Die unterschiedlichen Spektren werden dann vor oder am jeweiligen Detektor oder Detektorbereich durch mindestens einen Filter für die Röntgenstrahlung erzeugt. Es entstehen so Bereiche unterschiedlicher Spektren, die von den benachbarten Detektorbereichen eines einzelnen Detektors oder gegebenenfalls auch mehrerer Detektoren für jeweils ein Spektrum, aufgenommen werden und hiervon unterschiedliche Aufnahmen bzw. Bilder erzeugt werden.

Erfindungsgemäß ist es vorgesehen, mehrere elektromagnetische Strahlungen unterschiedlicher Spektren durch beispielsweise mehrere Strahlungserzeuger zu generieren. Dann wird der jeweils zu inspizierenden textile Gegenstand elektromagnetischer Strahlung unterschiedlicher Spektren ausgesetzt. Das geschieht vorzugsweise nacheinander. Anschließend wird vom mindestens einen Detektor vorzugsweise aufeinanderfolgend, gegebenenfalls aber auch simultan, für jedes erzeugte unterschiedliche Spektrum die jeweilige elektromagnetische Strahlung erfasst. Insbesondere wird von jedem Spektrum nacheinander eine Aufnahme gemacht und diese Aufnahmen zeitgleich nebeneinander beispielsweise mindestens einer Person dargestellt. Diese mindestens eine Person kann dann durch Vergleich der Bilder sämtliche Fremdkörper erkennen, und zwar gegebenenfalls auch verschiedene Bilder.

Denkbar ist es bei einer bevorzugten Weiterbildung des Verfahrens aber auch, die Bilder elektronisch durch eine Art Verrechnung so zu überlagern, dass nur ein Bild erzeugt wird, auf dem alle für die verschiedenen Spektren erfassten Fremdkörper erscheinen. Das lässt eine besonders einfache, schnelle und zuverlässige Ermittlung aller Fremdkörper im oder am jeweils inspizierten textilen Gegenstand durch insbesondere mindestens eine Person zu.

Erfindungsgemäß ist es vorgesehen, die Ermittlung von Fremdkörpern in oder an den textilen Gegenständen mit elektromagnetischen Strahlen verschiedener Spektren durchzuführen, wobei es sich den elektromagnetischen Strahlen um Röntgenstrahlen handelt. Es können so automatisch pro Zeiteinheit sehr viel mehr textile Gegenstände inspiziert werden, als das manuell möglich wäre. Außerdem ist es vorgesehen, dass die Inspektion der textilen Gegenstände mit elektromagnetischen Strahlen an einer solchermaßen von Personen beabstandeten und/oder abgeschirmten Stelle erfolgt, dass eine Strahlenbelastung der Person unter einem maximal zulässigen Grenzwert liegt. Dadurch kann das Inspizieren mit Röntgenstrahlen durchgeführt werden, vor denen Personen geschützt werden müssen.

Das Verfahrens sieht es vor, dass mindestens die von der wenigstens einen elektromagnetischen Strahlung sichtbar gemachten Fremdkörper durch wenigstens eine bildgebende Einrichtung oder wenigstens einen Detektor, die bevorzugt mindestens einen Teil des Wäschestücks abbilden, aufgenommen und dabei für jedes einzelne Wäschestück erfasste Daten, vorzugsweise Bilddaten, mindestens eines Bildes oder wenigstens einer Abbildung in wenigstens einem elektronischen Speicher abgespeichert werden.

Bevorzugt werden nur von solchen textilen Gegenständen Bilddaten abgespeichert, bei denen von den elektromagnetischen Strahlen mindestens ein Fremdkörper sichtbar gemacht worden ist. Alternativ ist es vorgesehen, dass die von der mindestens einen bildgebenden Einrichtung oder wenigstens einem Detektor aufgenommenen Bilddaten als Bild des jeweiligen textilen Gegenstands mit dem mindestens einen erfassten Fremdkörper visuell dargestellt werden. Das kann beispielsweise durch Abrufen der Bilddaten aus dem Speicher mit darin abgespeicherten Bilddaten geschehen.

Anhand der Bilddaten lässt sich ersehen, wie viele und welche Fremdkörper sich im oder am jeweiligen textilen Gegenstand befinden. Da die Bilddaten vorzugsweise von der Flachseite des jeweiligen hängenden textilen Gegenstands aufgenommen werden, geben die Bilddaten auch Auskunft darüber, wo sich der jeweilige Fremdkörper im oder am textilen Gegenstand befindet. Anhand der gewonnenen Informationen, nämlich Anzahl, Art und Position der Fremdkörper, lässt sich gezielt und somit zeitsparend der jeweilige Fremdkörper vom mindestens einen solchen aufweisenden textilen Gegenstand trennen bzw. entfernen. Durch das Abspeichern eines oder jedes aufgenommenen Röntgenbildes des jeweiligen durch Röntgenstrahlen durchstrahlten und/oder durchsichtig gemachten textilen Gegenstands und die dabei erfolgte Sichtbarmachung der daran oder darin befindlichen Fremdkörper erfolgt eine Visualisierung oder Anzeige der Fremdkörper hinsichtlich ihrer Art, Größe und Positionierung an bzw. im jeweiligen textilen Gegenstand, und zwar vorzugsweise unter Zuordnung zum jeweiligen textilen Gegenstand.

Durch das Abspeichern der Bilddaten eines oder mehrerer Bilder bzw. Aufnahmen des jeweiligen textilen Gegenstands können diese zu einem beliebigen Zeitpunkt abgerufen und/oder mit anderen Bilddaten verglichen werden. So kann auch eine Dokumentation erfolgen und/oder anhand abgespeicherter Referenzbilder eine Datenbank typischer Fremdkörper erzeugt werden. Durch Vergleich der abgespeicherten Fremdkörper in der Datenbank mit aktuellen Bildern kann dann automatisch auf elektronischem Wege ermittelt werden, um welche Art Fremdkörper es sich aktuell handelt.

Die Bilddaten werden visuell an einem solchen Ort dargestellt, an dem keine Strahlenbelastung vom Inspektionsvorgang vorhanden ist oder zumindest die Strahlenbelastung unterhalb des maximalen Grenzwerts liegt. Dann kann mindestens eine Person ungefährdet durch die zur Inspektion bzw. Visualisierung von Fremdkörpern dienenden elektromagnetischen Strahlen den oder die Fremdkörper vom jeweiligen textilen Gegenstand anhand des mindestens einen ihr dargebotenen Bildes gezielt und rasch trennen und vor allem keinen Fremdkörper übersehen oder vergessen. Zu diesem Zweck kann beispielsweise die Anzahl der beim jeweiligen textilen Gegenstand festgestellten Fremdkörper der Person zusätzlich als Ziffer angezeigt werden, so dass sie genau weiß, wie viele Fremdkörper sie entfernen muss.

Vorzugsweise erfolgt die Inspektion der textilen Gegenstände durch Detektion von eventuell darin vorhandenen Fremdkörpern mit solchen elektromagnetischen Strahlen, die geeignet sind, die Fremdkörper, und zwar nicht nur metallische Fremdkörper, sondern auch andere nichtmetallische Fremdkörper, in den textilen Gegenständen sichtbar zu machen. Infolge der Sichtbarmachung von eventuellen Fremdkörpern in textilen Gegenständen kann eine Vorsortierung erfolgen nach textilen Gegenständen ohne Fremdkörper und textilen Gegenstände mit jeweils mindestens einem Fremdkörper. Nur diese textilen Gegenstände brauchen dann anschließend zur Befreiung von den Fremdkörpern bereitgestellt zu werden.

Bei einer bevorzugten Ausgestaltung des Verfahrens werden die textilen Gegenstände im mindestens teilweise frei herunterhängenden Zustand einer gleiche oder auch unterschiedliche Fremdkörper detektierenden elektromagnetischen Strahlung bzw. Strahlungen ausgesetzt. Vorzugsweise erfolgt das bei an einem Stetigförderer hängenden und/oder an bzw. auf einem Bügel hängenden Zustand der textilen Gegenstände. Durch die Inspektion der textilen Gegenstände im mindestens teilweise hängenden Zustand sind die textilen Gegenstände ausreichend gestreckt bzw. ausgebreitet, um Fremdkörper in und/oder an demselben durch elektromagnetische Strahlung automatisch detektieren zu können.

Vorzugsweise erfolgt die Detektion der Fremdkörper an einzelnen textilen Gegenständen. Demzufolge wird jeder textile Gegenstand für sich individuell auf das Vorhandensein mindestens eines Fremdkörpers inspiziert. Je nachdem, wie groß der Durchsatz zu inspizierender textiler Gegenstände pro Zeiteinheit ist, können zwei oder auch mehr als zwei textile Gegenstände gleichzeitig inspiziert werden. Das geschieht vorzugsweise, indem jeder textile Gegenstand für sich inspiziert wird.

Eine besonders vorteilhafte Ausgestaltungsmöglichkeit des Verfahrens sieht es vor, dass die Detektion der Fremdkörper in oder an den textilen Gegenständen im Bereich einer Speicherstrecke der textilen Gegenstände erfolgt. Alternativ oder zusätzlich kann es vorgesehen sein, die Detektion der Fremdkörper in einer Ebene, insbesondere Etage, vorzunehmen, die sich über oder unter einer Ebene bzw. Etage befindet, auf der Personen die Wäschestücke handhaben und/oder bearbeiten. Durch die Inspektion der textilen Gegenstände nach Fremdkörpern in einer anderen Ebene bzw. anderem Niveau findet das Inspizieren der textilen Gegenstände fernab von manuellen Arbeiten an den textilen Gegenständen statt. Dadurch ist sichergestellt, dass die Personen mit einem ausreichenden Abstand zur Stelle, an der die textilen Gegenstände mit Röntgenstrahlen inspiziert werden, tätig sind. Dieser Abstand ist bevorzugt so groß, dass die Personen keiner nennenswerten Strahlenbelastung mehr ausgesetzt sind, zumindest aber die Strahlenbelastung unter dem maximal zulässigen Grenzwert liegt. Dadurch ist sichergestellt, dass die zur Inspektion der textilen Gegenstände verwendeten Röntgenstrahlung zu keiner oder mindestens keiner unzulässig hohen Strahlenbelastung führen, die sich auf Personen schädlich auswirken können.

Die Detektion von eventuellen Fremdkörpern in bzw. an den textilen Gegenständen findet bevorzugt nach dem Anhängen derselben an den Stetigförderer oder nach dem Aufhängen des textilen Gegenstands auf den Bügel oder Anhängen an den Bügel statt. Durch das Anhängen der textilen Gegenstände an den Förderer oder Aufhängen auf Bügel, insbesondere Transportbügel, hängen die textilen Gegenstände zumindest größtenteils vom Förderer bzw. Bügel herunter. Das begünstigt und ermöglicht die Inspektion mit elektromagnetischen Strahlen. Indem diese Inspektion im Anschluss an das Anhängen der textilen Gegenstände an den Förderer oder Aufhängen auf Transportbügel stattfindet, werden die textilen Gegenstände vor nachfolgenden Bearbeitungen auf Fremdkörper untersucht, so dass festgestellte Fremdkörper entfernt werden können, bevor eine anschließende Behandlung der textilen Gegenstände, insbesondere Sortieren, Nassbehandeln oder dergleichen, stattfindet. Dadurch können die festgestellten und entfernten Fremdkörper der nachfolgenden Behandlung der textilen Gegenstände und daran beteiligten Personen keinen Schaden mehr anrichten.

Es ist auch denkbar, nach dem Entfernen des mindestens einen Fremdkörpers durch die jeweilige Person den betreffenden Gegenstand erneut zu inspizieren, um zu kontrollieren, ob wirklich alle Fremdkörper von der Person entfernt worden sind. Diese Inspektion erfolgt genauso wie zuvor im Zusammenhang mit einer Sichtbarmachung von Fremdkörpern beschriebenen Verfahrensweise. Vorzugsweise dient dazu eine separate Einrichtung, die auch an einem anderen Ort, insbesondere einer anderen Etage, angeordnet sein kann, wo sichergestellt ist, dass die an den textilen Gegenständen tätigen Personen keiner Strahlenbelastung oder jedenfalls keiner über dem zulässigen Grenzwert liegenden Strahlenbelastung ausgesetzt sind.

Eine andere vorteilhafte Ausbildungsmöglichkeit des Verfahrens sieht es vor, die textilen Gegenstände, vorzugsweise jeweils nur einen einzelnen textilen Gegenstand oder nur wenige, zum Beispiel zwei oder drei, textile Gegenstände, in einer mindestens größtenteils geschlossenen Kammer mit Röntgenstrahlen zum Zwecke der Inspektion zu beaufschlagen bzw. zu bestrahlen. Diese Kammer bietet eine Abschirmung der in ihrem Inneren auf den mindestens einen textilen Gegenstand gerichteten elektromagnetischen Strahlung. Dadurch tritt keine oder höchstens eine verringerte Strahlung aus der Kammer aus. Auf diese Weise kann die Inspektion der textilen Gegenstände mit elektromagnetischen Strahlen in geringerer Entfernung von mit Personen besetzten Arbeitsplätzen erfolgen, als das bei einer Inspektion der textilen Gegenstände ohne eine solche Abschirmung in mindestens einer Kammer möglich wäre. Alternativ zur Kammer oder gegebenenfalls auch zusätzlich kann es vorgesehen sein, mindestens eine Strahlenquelle und/oder mindestens einen Strahlungsgenerator abzuschirmen, indem dieser beispielsweise in einem - abgesehen vom Strahlungsaustritt - abgeschirmten oder abschirmenden Gehäuse angeordnet ist.

Gemäß einer vorteilhaften Weiterbildung des Verfahrens wird die Länge der textilen Gegenstände, also der Abstand zwischen dem oberen Ende und dem unteren Ende, für die Inspektion mit Röntgenstrahlen reduziert. Dadurch braucht nur eine kleinere Fläche des jeweiligen textilen Gegenstands mit elektromagnetischer Strahlung beaufschlagt und/oder von der elektromagnetischen Strahlung durchleuchtet zu werden. Das reduziert die erforderliche Strahlendosis. Vorzugsweise erfolgt die Reduzierung der Länge des jeweiligen textilen Gegenstands wenigstens im Bereich der Kammer, in der der jeweilige textile Gegenstand der elektromagnetischen Strahlung ausgesetzt wird.

Weiterhin ist es bevorzugt denkbar, die Länge des jeweiligen textilen Gegenstands zu reduzieren, indem ein Unterteil desselben auf einem Boden der Kammer oder einer ortsfesten Führungsbahn und/oder einem Gurtförderer abgestützt wird. Die Verkürzung der Länge des jeweiligen textilen Gegenstands findet dann quasi durch eine "Stauchung" mindestens eines Teil des Gegenstands statt, wobei ein Teil, vorzugsweise ein unterer Teil, des jeweiligen textilen Gegenstands sich zur Seite bewegt und/oder hochgedrückt wird.

Gemäß einer vorteilhaften Weiterbildung des Verfahrens erfolgt vor der Inspektion automatisch eine Identifikation der mindestens einen Datenträger aufweisenden textilen Gegenstände.

Insbesondere ist es vorgesehen, bei textilen Gegenständen, die mit einem Datenträger versehen sind, vor der Inspektion dieser textilen Gegenstände die Datenträger auszulesen. Durch die dabei gewonnenen Daten können Informationen über die betreffenden zu inspizierenden textilen Gegenstände erhalten werden. Dabei handelt es sich bevorzugt um die Ermittlung der Art der textilen Gegenstände. Daraus kann abgeleitet werden, ob die textilen Gegenstände etwas aufweisen, was als Fremdkörper angesehen werden könnte, ohne ein solcher zu sein, beispielsweise metallische Bestandteile, Reißverschlüsse oder Knöpfe.

Nachdem die Datenträger der textilen Gegenstände, und zwar aller textiler Gegenstände oder nur solcher textiler Gegenstände, die Datenträger aufweisen, ausgelesen worden sind, erfolgt die Inspektion der textilen Gegenstände. Dabei wird anhand der ausgelesenen Daten eine Art Kategorisierung der textilen Gegenstände vorgenommen. Diese Kategorisierung erfolgt nach der Art der textilen Gegenstände und/oder das Vorhandensein von Gegenständen, beispielsweise metallischen und auch nichtmetallischen Knöpfen oder Reißverschlüssen, an den textilen Gegenständen. Bei der Auswertung des Ergebnisses der Inspektion, insbesondere der dabei detektierten Fremdkörper, können die anhand der ausgelesenen Daten gefundenen Informationen berücksichtigt werden. Wenn zum Beispiel bei der Inspektion eine Reihe übereinanderliegender, nicht textiler Gegenstände festgestellt wird, können diese als Fremdkörper unberücksichtigt bleiben, weil anhand der vorher ausgelesenen Daten des Datenträgers des jeweiligen textilen Gegenstands davon auszugehen ist, dass es sich hierbei um Knöpfe handelt, die keine Fremdkörper darstellen.

Bevorzugte Ausführungsbeispiele der Erfindung werden nachfolgend anhand der Zeichnung näher erläutert. In dieser zeigen.
- Fig. 1: eine Seitenansicht eines Teils einer Vorrichtung mit einer Detektionseinrichtung für textile Gegenstände,
- Fig. 2: einen mittigen Längsschnitt durch die Detektionseinrichtung der Fig. 1,
- Fig. 3: eine perspektivische Ansicht der Detektionseinrichtung der Fig. 2,
- Fig. 4: ein zweites Ausführungsbeispiel eines Teils einer Vorrichtung zur Inspektion textiler Gegenstände im Bereich der Detektionseinrichtung, und
- Fig. 5: ein von der Detektionseinrichtung aufgenommenes Bild eines ausgebreiteten textilen Gegenstands mit drei bei der Inspektion detektierten Fremdkörpern.

Die Figuren zeigen verschiedene Ausführungsbeispiele von einem sich auf das erfindungsgemäße Verfahren zum Inspizieren von textilen Gegenständen, insbesondere Formteilen und Flachwäsche, beziehenden Teil einer Vorrichtung.

Bei der Vorrichtung kann es sich um ein durch mindestens einen Teil einer Wäscherei erstreckendes Transportsystem 10 für textile Gegenstände handeln. Bei den textilen Gegenständen handelt es sich im vorliegenden Ausführungsbeispiel um Formteile, nämlich Arbeitskittel 11, ohne dass die Erfindung hierauf beschränkt sein soll. Wenn demnach im Folgenden nur "Arbeitskittel 11" genannt sind, schließt das auch andere textile Gegenstände mit ein, ist also als "Arbeitskittel 11 und andere textile Gegenstände" zu verstehen. Es können vom Transportsystem 10 gleichzeitig mehrere Arbeitskittel 11, aber auch unterschiedliche textile Gegenstände, beispielsweise Arbeitskittel 11, Hosen, T-Shirts, Hemden, Jacken oder auch Flachwäscheteile, die hintereinander und/oder aufeinander , vorzugsweise beabstandend, folgend in Transportrichtung 12 durch die Wäscherei transportiert werden.

Das Transportsystem 10 verfügt über einen vorzugsweise umlaufenden, ortsfesten Förderstrang 13 mit zum Beispiel wenigstens einer Schiene. Der Förderstrang 13 weist einen den gewünschten Transportweg der Arbeitskittel 11 oder dergleichen durch die Wäscherei entsprechenden Verlauf auf und ist vorzugsweise als Stetigförderer ausgebildet.

Am Förderstrang 13 können vorzugsweise beabstandete Laufwagen oder Mitnehmer verfahrbar sein. Die Laufwagen oder Mitnehmer werden mindestens entlang ausgewählter Abschnitte des Förderstrangs 13 von einem demselben zugeordneten umlaufend antreibbaren Treibstrang, beispielsweise einer Kette, in Transportrichtung 12 längs des Förderstrangs 13 bewegt. Dies kann kontinuierlich oder auch diskontinuierlich geschehen. Entlang bestimmter Abschnitte des Förderstrangs 13 können die Arbeitskittel 11 frei und schwerkraftbedingt weitertransportierbar sein. Dazu weisen solche Abschnitte des Förderstrangs 13 einen entsprechend geneigten Verlauf auf.

Unter dem jeweiligen Laufwagen oder auf dem Förderstrang 13 aufgehängt sind Transportbügel 15. Die Transportbügel 15 können mit dem dazugehörigen Laufwagen dauerhaft verbunden oder auch abnehmbar angehängt sein. Jeder Transportbügel 15 verfügt im gezeigten Ausführungsbeispiel über einen Bügelhaken 16, ein am unteren Ende desselben angeordnetes Bügelgehäuse 17 und zwei gegenüberliegenden Seiten des Bügelgehäuses 17 zugeordnete, vorzugsweise gleiche Bügelarme 18. Auf den vom Bügelgehäuse 17 auf gegenüberliegenden Seiten seitlich abstehenden Bügelarmen 18 ist jeweils ein Arbeitskittel 11 aufhängbar, so dass die Arbeitskittel 11 vom Transportbügel 15 herunterhängen. Die Bügelarme 18 des Transportbügels 15 können zum automatischen Abbügeln der Arbeitskittel 11 einklappbar sein. Denkbar ist es auch, die Transportbügel 15 mit ihren Bügelhaken 16 auf dem Förderstrang aufzuhängen, der dann keine Laufwagen, sondern lediglich Mitnehmer aufzuweisen braucht.

Im gezeigten Ausführungsbeispiel weisen die Transportbügel 15 unter ihren Bügelgehäusen 17 noch jeweils eine Klammer 14 für zum Beispiel Hosen oder Flachwäschestücke auf.

Der Förderstrang 13 verfügt am Anfang über einen sich in einer unteren Ebene befindenden, vorzugsweise horizontal verlaufenden Beladeabschnitt 19. Dort ist von mindestens einer Person 20 jeweils ein Arbeitskittel 11 auf einen Transportbügel 15 aufhängbar. In Transportrichtung 12 gesehen schließt sich an den Beladeabschnitt 19 eine Steigstrecke 21 des Förderstrangs 13 an. Entlang der Steigstrecke 21 werden die auf den Transportbügeln 15 hängenden Arbeitskittel 11 in eine zweite, höhere Ebene hochtransportiert. In dieser zweiten höheren Ebene kann sich mindestens ein Speicher 22 für eine Vielzahl von auf Transportbügeln 15 hängenden Arbeitskitteln 11 befinden. Zumindest im Bereich des Speichers 22 befinden sich die Arbeitskittel 11 quer zur Transportrichtung orientiert in dichter Aufeinanderfolge, so dass eine Vielzahl von Arbeitskitteln 11 im Speicher 22 Aufnahme findet. Im Bereich des Speichers 22 verfügt der Förderstrang 13 vorzugsweise über einen mäanderförmigen Verlauf mit mehreren vorzugsweise parallelen Abschnitten, wobei benachbarte Abschnitte an ihren Enden zum durchgehenden Förderstrang 13 verbunden sind. Die Anzahl der Abschnitte richtet sich nach der Größe des Speichers 22, also die Anzahl der im Speicher 22 unterbringbaren Arbeitskittel 11. Am Ende des Speichers 22 weist der Förderstrang 13 eine Gefällestrecke 23 auf, längs derer die Arbeitskittel 11 von der höheren Ebene im Bereich des Speichers 22 zurück in die untere Ebene gelangen, in der sich der Beladebabschnitt 19 befindet. In dieser unteren Ebene folgt auf die Gefällestrecke 23 eine vorzugsweise horizontale Fremdkörperentnahmestrecke 24. An der Fremdkörperentnahmestrecke 24 sind von mindestens einer weiteren Person 25 Fremdkörper 37 aus den solche aufweisenden Arbeitskitteln 11 manuell entfernbar.

Im Bereich des sich in einer höheren Etage befindenden Speichers 22, gegebenenfalls davor oder dahinter, ist wenigstens eine Detektionseinrichtung 26 zur Inspektion der Arbeitskittel 11 auf mindestens einen darin oder daran sich befindenden Fremdkörper 37 vorgesehen. Die Detektionseinrichtung 26 ist zur automatischen berührungslosen Ermittlung, insbesondere bildhaften Erfassung und/oder Darstellung, von Fremdkörpern 37 in den Arbeitskitteln 11 mittels Bestrahlung und/oder Durchleuchtung der Arbeitskittel 11 mit elektromagnetischen Strahlen ausgebildet. Bei den elektromagnetischen Strahlen handelt es sich um Röntgenstrahlung.

Die Detektionseinrichtung 26 ist im gezeigten Ausführungsbeispiel ausgebildet, um jeweils mindestens einen einzelnen an einem Transportbügel 15 des Transportsystems 10 hängenden Arbeitskittel 11 auf das Vorhandensein mindestens eines Fremdkörpers 37 zu inspizieren. Dazu verfügt die Detektionseinrichtung 26 über mindestens eine Strahlenquelle 27, die auch als Strahlungsquelle bezeichnet werden kann, zur Erzeugung von Röntgenstrahlen zur Sichtbarmachung von Fremdkörpern 37 in oder an den Arbeitskitteln 11. Die mindestens eine Strahlenquelle kann abgeschirmt sein. Außerdem verfügt die Detektionseinrichtung 26 über mindestens einen Detektor 28, der den textilen Gegenstand, insbesondere Arbeitskittel 11, wenigstens aber einen solchen Teil desselben, der üblicherweise Fremdkörper aufweisen kann, beim Röntgen desselben bildlich erfasst und/oder abscannt. Der mindestens eine Detektor 28 kann deshalb auch als Röntgenscanner bezeichnet werden, der auch eventuell beim Röntgen sichtbar gemachte bzw. gewordene Fremdkörper 37 erfasst, insbesondere bildlich erfasst und/oder abscannt. Der Detektor 28 weist bevorzugt mindestens einen Zeilendetektor auf oder es handelt sich beim Detektor 28 um mindestens einen Zeilendetektor, der zeilenweise ein Bild vom jeweiligen inspizierten Arbeitskittel 11 und den gegebenenfalls mindestens einen darin enthaltenen Fremdkörper 37 erzeugt. Alternativ kann der Detektor 28 auch mit einer das von der Strahlenquelle 27 erzeugte Bild des Arbeitskittels 11 mit der gegebenenfalls einen Fremdkörper 37 aufnehmenden Kamera versehen und/oder als Kamera ausgebildet sein.

Im gezeigten Ausführungsbeispiel ist die Detektionseinrichtung 26, also die Strahlenquelle 27 und der Detektor 28, die zusammen insbesondere einen Röntgenscanner bilden, in einer größtenteils geschlossenen Kammer 29 angeordnet. Die Kammer 29 ist so ausgebildet, insbesondere hinsichtlich ihres Materials, dass sie den Austritt der von der Strahlenquelle 27 erzeugten Röntgenstrahlen, mindestens größtenteils unterbindet. Zusätzlich zur Kammer 29 kann eine Abschirmung der mindestens einen Strahlenquelle 27 vorgesehen sein, aber auch eine Abschirmung die Kammer 29 ersetzen.

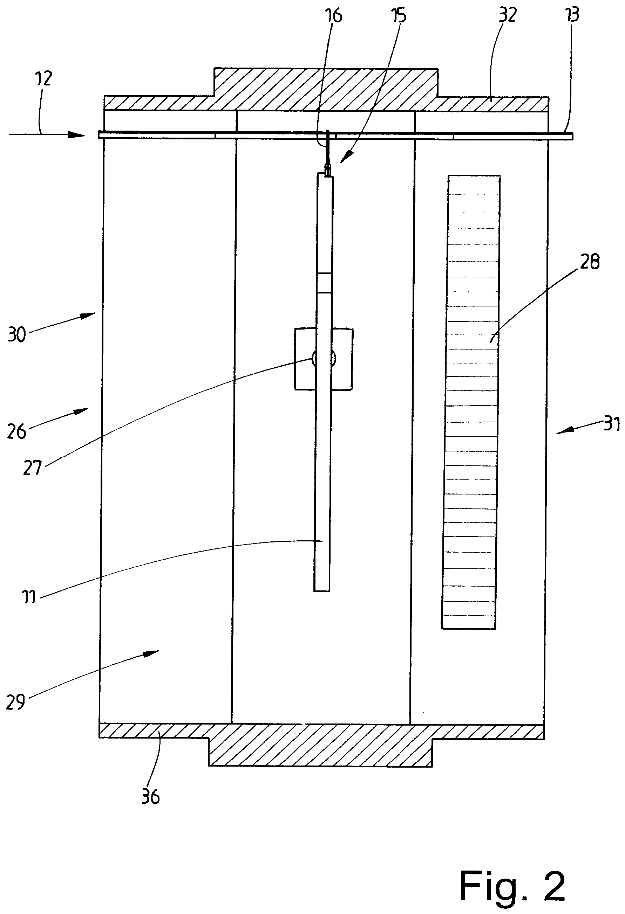

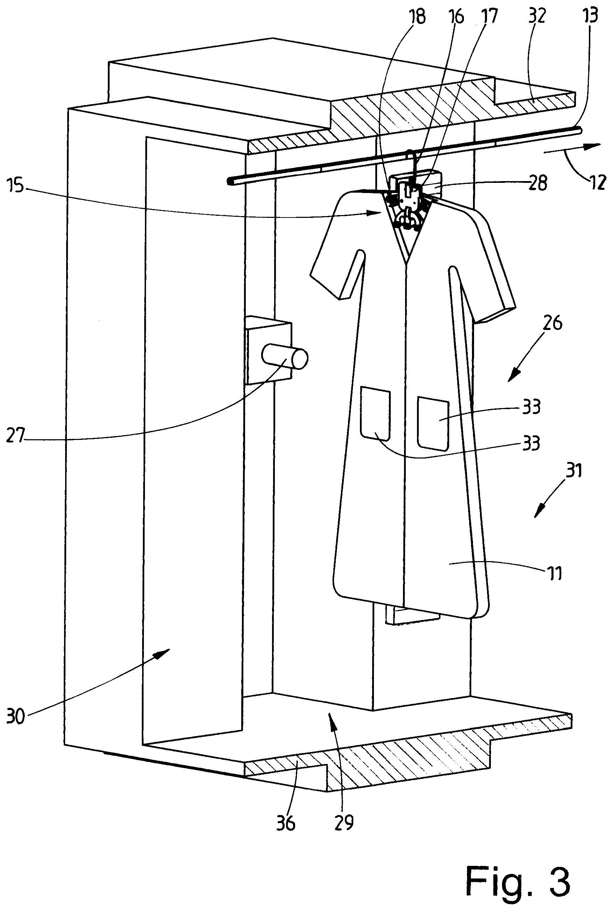

Die Kammer 29 ist weiterhin so ausgebildet, dass durch diese die Arbeitskittel 11 einzeln nacheinander von dem Transportsystem 10 hindurchförderbar sind. Dazu verfügt die Kammer 29 über eine Einlassöffnung 30 und eine Auslassöffnung 31, die gegebenenfalls verschließbar sein können. Die Einlassöffnung 30 und die Auslassöffnung 31 folgen in Transportrichtung 12 aufeinander, sind also am Anfang und am Ende der Kammer 29 angeordnet. Im gezeigten Ausführungsbeispiel werden die Arbeitskittel 11 mit einer quer zur Transportrichtung 12 verlaufenden Orientierung durch die Kammer 29 transportiert. Die Transportrichtung des Transportsystems 10 verläuft dadurch längs der Flächennormalen zu den (großen) Flachseiten der Arbeitskittel 11. Damit dann der mindestens eine Detektor 28, insbesondere der in den Fig. 2 und 3 dargestellte Zeilendetektor, der Detektionseinrichtung 26 bei dieser Orientierung der Arbeitskittel 11 eine Flachseite bildlich erfassen und/oder abscannen kann, ist der Detektor 28 von einer in den Fig. 2 und 3 gezeigten Ruhestellung neben dem Arbeitskittel 11 herausfahrbar und/oder verschwenkbar. Der Detektor 28 kann eine Flachseite des betreffenden Arbeitskittels 11 abscannen und hiervon Bilddaten erzeugen. Der Detektor 28 wird nur kurzzeitig zur Erzeugung eines Bilds vom Arbeitskittel 11 mit durch Röntgen sichtbar gemachten Fremdkörpern 37 mit Abstand vor die Flachseite des Arbeitskittels 11 gefahren und danach sofort wieder in seine Ausgangsstellung zurückgefahren zur Ermöglichung des Hindurchtransportierens des jeweiligen Arbeitskittels 11 durch die Kammer 29. Ebenso ist die mindestens eine Strahlenquelle 27, als Röntgenstrahlenquelle, aus der in den Fig. 2 und 3 gezeigten Stellung herausfahrbar und/oder verschwenkbar, in eine Arbeitsstellung, bei der sie auf die vom Detektor 28 abgewandte Flachseite des Arbeitskittels 11 gerichtet ist. Alternativ ist es denkbar, den Detektor 28, insbesondere Zeilendetektor, schräg zur Transportrichtung 12, beispielsweise unter einem Winkel von 45°, neben dem Transportweg der Arbeitskittel 11 durch die Kammer 29 anzuordnen. Dann braucht der Detektor 28 zum Aufnehmen eines Bildes vom geröntgten Arbeitskittel 11 nicht vor- und zurückgefahren zu werden.

Außerdem ist es auch denkbar, die Arbeitskittel 11 mit einer quer zur Transportrichtung 12 verlaufenden Orientierung durch die Kammer 29 zu transportieren. Dann erübrigt sich auch ein Verfahren bzw. eine schräge Anordnung des Detektors 28, weil dieser dann stets auf eine Flachseite des Arbeitskittels 11 blickt bzw. gerichtet ist.

Der Förderstrang 13 des Transportsystems 10 ist bei den gezeigten Ausführungsbeispielen durch die Kammer 29 geführt, und zwar dicht unter der Decke 32 desselben entlang. Dann verläuft der Förderstrang 13 im Bereich der Kammer 29 durch seine gegebenenfalls verschließbare Einlassöffnung 30 und Auslauföffnung 31 hindurch zusammen mit dem jeweiligen Arbeitskittel 11 und dem diesen tragenden Transportbügel 15. Es ist aber auch denkbar, den Förderstrang 13 des Transportsystems 10 über die Decke 32 der Kammer 29 hinwegzuführen. Der Förderstrang 13 verläuft dann außerhalb der Kammer 29. Der Bügelhaken 16 des Transportbügels 15 erstreckt sich dabei durch die Decke 32 der Kammer 29. Zu diesem Zweck wäre die Decke 32 der Kammer 29 mit einem in Transportrichtung 12 durchgehenden schmalen Schlitz zu versehen.

Im Inneren der Kammer 29 sind die mindestens eine Strahlenquelle 27 und dieser gegenüberliegend der Detektor 28 angeordnet, der hier als vertikaler balkenartiger Zeilendetektor ausgebildet ist. Am Detektor 28 laufen die zu inspizierenden Arbeitskittel 11 durch die Kammer 29, so dass der jeweilige Arbeitskittel 11 mit Röntgenstrahlen der Strahlenquelle 27 beaufschlagt werden kann. Der Detektor 28, insbesondere Zeilendetektor, ist derart relativ zum jeweiligen Arbeitskittel 11 angeordnet oder bewegbar, dass eine Flachseite des Arbeitskittels 11 mindestens größtenteils abgescannt werden kann zur Erzeugung eines Röntgenscans des Arbeitskittels 11. Der mindestens eine Detektor 28 ist so bemessen und angeordnet, dass er nicht das Transportsystem 10 und/oder den Transportbügel 15 bestrahlt und/oder beeinflusst, sondern nur maximal den jeweiligen textilen Gegenstand.

Die vom Detektor 28 aufgenommenen Bilddaten werden bevorzugt in einer Bilddatei abgespeichert. Insbesondere kann es vorgesehen sein, dem Arbeitskittel 11 oder einem sonstigen textilen Gegenstand ein identifizierendes Merkmal, zum Beispiel eine Nummer, zuzuordnen.

Abweichend zur Darstellung in der Fig. 2 ist es denkbar, den als mindestens einen Zeilendetektor ausgebildeten Detektor 28 in einer horizontalen Längserstreckung anzuordnen. Ein solcher Zeilendetektor kann kürzer sein als beim Ausführungsbeispiel der Fig. 2. Der Zeilendetektor wird vorzugsweise vor einer Flachseite des textilen Gegenstands auf einer vertikalen Bahn hoch- und/oder heruntergefahren, wobei es zu einem flächigen Abscannen des textilen Gegenstands kommt. Es kann reichen, den horizontalen Zeilendetektor nur so weit vor oder gegebenenfalls auch hinter dem zu detektierenden Arbeitskittel 11 oder einen sonstigen textilen Gegenstand auf und ab zu verfahren, dass derjenige Teil oder Bereich des textilen Gegenstands abgescannt wird, indem sich typischerweise Fremdkörper befinden.

Der jeweilige Detektor 28, insbesondere der vertikale Zeilendetektor (Fig. 2) oder der horizontale Zeilendetektor, können "fliegend" angeordnet sein. Das geschieht zweckmäßigerweise so, dass mindestens der wenigstens eine Detektor 28, gegebenenfalls auch die gesamte Detektionseinrichtung 26, synchron mit dem vom Transportsystem in Transportrichtung 12 weitertransportierten Arbeitskitteln 11 mitbewegt werden. Das ermöglicht eine Detektion von Fremdkörpern während des Weitertransports der Arbeitskittel 11 oder sonstiger textiler Gegenstände. So kann die Detektion von Fremdkörpern während des kontinuierlichen Weitertransports der textilen Gegenstände, insbesondere Arbeitskittel 11, erfolgen.

Es ist ein weiteres alternatives Ausführungsbeispiel der Vorrichtung denkbar, bei dem die Detektion von Fremdkörpern in den oder an den textilen Gegenständen auf einem die Strahlen der wenigstens einen Detektionseinrichtung 26 nicht beeinflussenden Transportband erfolgt. Die textilen Gegenstände werden dann im flach auf dem Transportband liegenden Zustand inspiziert durch Beaufschlagung mit elektromagnetischer Strahlung. Das nach Art eines Gurtförderers ausgebildete Transportband transportiert nacheinander flach auf demselben liegende textile Gegenstände an der Detektionseinrichtung 26 vorbei. Mit dieser Vorrichtung ist eine kontinuierliche Detektion der textilen Gegenstände hinsichtlich Fremdkörper möglich. Außerdem lassen sich die flach auf dem Transportband liegenden textilen Gegenstände sehr vorteilhaft und vor allem vollflächig auf Fremdkörper mit elektromagnetischen Strahlen inspizieren, insbesondere lückenlos abscannen.

An der Fremdkörperentnahmestrecke 24 ist der dort tätigen Person 25 ein Monitor 34 zugeordnet. Auf dem Monitor 34 werden der Bedienungsperson 25 die von der Detektionseinrichtung 26 aufgenommenen Bilddaten, erfindungsgemäß der Röntgenscan, visuell für den jeweiligen momentan in ihm vorbeilaufenden Arbeitskittel 11 angezeigt. Vorzugsweise werden nur von solchen an der Person 25 vorbeitransportierten Arbeitskitteln 11 Bilddaten angezeigt, die mindestens einen Fremdkörper 37 aufweisen.

Arbeitskittel 11, bei denen die Detektionseinrichtung 26 keinen Fremdkörper 37 festgestellt hat, sind an der Person 25 im Bereich der Fremdkörperentnahmestrecke 24 kontinuierlich vorbeitransportierbar.

Der Abstand der Detektionseinrichtung 26 zu der sich am nächsten an derselben befindenden Personen, insbesondere den Personen 20 und 25, ist so gewählt, dass diese keiner nennenswerten Strahlenbelastung ausgesetzt werden, insbesondere höchstens einer Strahlenbelastung unterhalb des maximal zulässigen Grenzwerts ausgesetzt werden. Dies wird erreicht durch die Anordnung der Detektionseinrichtung 26, vorzugsweise zusammen mit dem Speicher 22, an einer anderen - hier höheren - Ebene oder Etage. Zusätzlich, so wie im gezeigten Ausführungsbeispiel oder auch alternativ, wird noch die Strahlenbelastung der Person auf ein Minimum reduziert durch die Anordnung der Detektionseinrichtung 26 in einer als Abschirmung dienenden Kammer 29. Gegebenenfalls kann zwischen der oberen und der unteren Etage noch eine in den Figuren nicht gezeigte Zwischendecke vorhanden sein. Beim Vorhandensein einer solchen Zwischendecke ist es denkbar, auf die Kammer 29 zu verzichten, weil dann die Zwischendecke die Funktion der Abschirmung übernimmt. Im Falle einer Abschirmung der elektromagnetischen Strahlung durch die Zwischendecke und/oder die Kammer 29 können Personen, insbesondere die Personen 20 und 25, gefahrlos räumlich dichter an der Detektionseinrichtung 26, insbesondere ihre Strahlenquelle 27, positioniert sein, um dort ihre Arbeiten auszuüben.

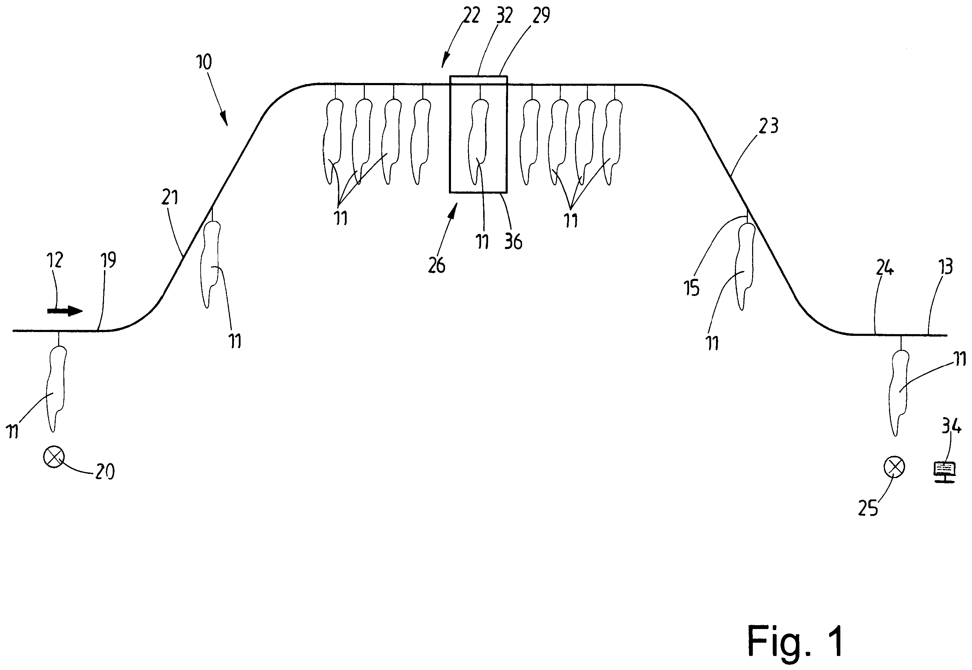

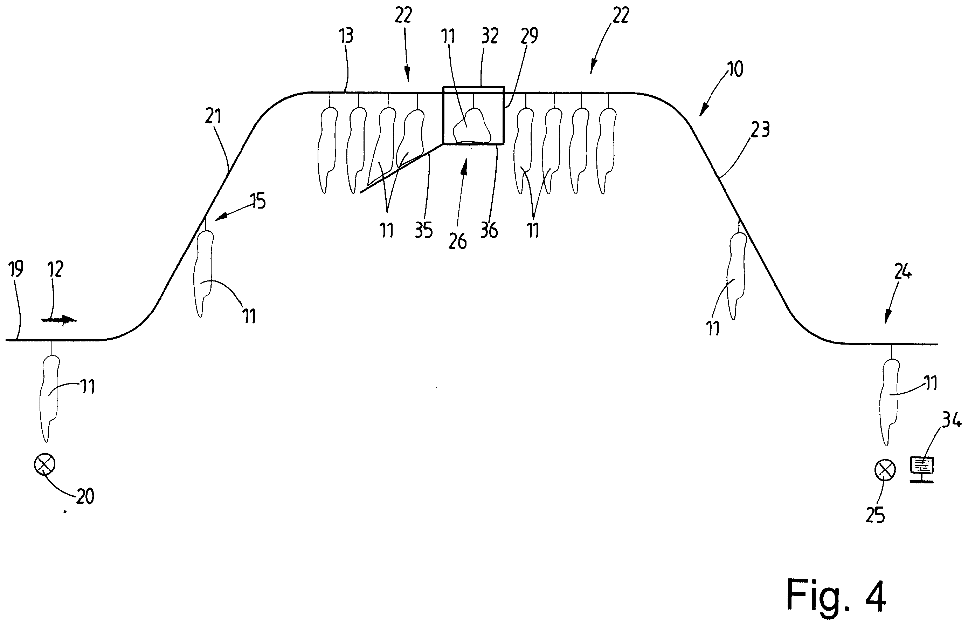

Die Fig. 4 zeigt ein alternatives Ausführungsbeispiel der Vorrichtung der Fig. 1. Bei diesem Ausführungsbeispiel ist der Kammer 29 eine feststehende Führungsbahn 35 für untere Bereiche der Arbeitskittel 11 zugeordnet. Diese Führungsbahn 35 verfügt in Transportrichtung 12 gesehen vor der Kammer 29 über einen schräg ansteigenden Anfangsabschnitt, der kurz vor der Einlassöffnung 30 der Kammer 29 in einen horizontalen Abschnitt übergeht. Dieser horizontale Abschnitt kann auf oder über einem Boden 36 der Kammer 29 angeordnet sein. Es ist aber auch denkbar, dass dieser horizontale Abschnitt der Führungsbahn 35 vom Boden 36 der Kammer 29 gebildet ist. Durch den schräg in Transportrichtung 12 gesehen ansteigenden Abschnitt der Führungsbahn 35 wird ein unterer Teil des Arbeitskittels 11 beim Entlanggleiten der Führungsbahn 35 nach hinten umgelenkt und dadurch die Länge des Arbeitskittels 11 reduziert, wodurch das Volumen der Kammer 29 verringert werden kann, indem diese eine Höhe aufweist, die geringer ist als die Längen der Arbeitskittel 11.

Alternativ kann statt der Führungsbahn 35 auch ein umlaufender Gurtförderer vorgesehen sein, dessen Verlauf dem Verlauf der Führungsbahn 35 entspricht. Dann werden die umgeknickten oder umgelenkten unteren Enden der Arbeitskittel 11 nicht gleitend über die Führungsbahn 35 gezogen, sondern vom Gurtförderer schlupffrei oder nur mit verringertem Schlupf beim Weitertransport des Arbeitskittels 11 durch das Transportsystem 10 synchron oder nahezu synchron weitertransportiert.

Das erfindungsgemäße Verfahren wird nachfolgend anhand der zuvor beschriebenen Vorrichtung beispielhaft erläutert:
Die in den Figuren als Arbeitskittel 11 dargestellten textilen Gegenstände, aber auch solche, die keine Arbeitskittel 11 sind, werden mit ausreichendem Abstand von Tätigkeiten ausführenden Personen, insbesondere den Personen 20 und 25, mit Röntgenstrahlen auf das Vorhandensein von Fremdkörpern 37 inspiziert. Das geschieht der vorliegender Erfindung durch Abscannen der Arbeitskittel 11 mit Röntgenstrahlen (Röntgen-Scan). Die Inspektion der Arbeitskittel 11 mit elektromagnetischer Strahlung erfolgt in einem solchen Abstand von den am nächsten an der dazu dienenden Detektionseinrichtung 26 tätigen Person, insbesondere den Personen 20 und 25, dass diese keiner nennenswerten Strahlenbelastung ausgesetzt werden. Zumindest ist aber die Strahlenbelastung der Personen 20, 25 soweit reduziert und/oder abgeschirmt, dass sie unterhalb festgelegter, insbesondere gesetzlicher, Grenzwerte und/oder Maximalwerte liegt.

Die Arbeitskittel 11 werden im gezeigten Ausführungsbeispiel nacheinander einzeln an Transportbügeln 15 hängend auf das Vorhandensein von Fremdkörpern 37 untersucht. Diese Untersuchung erfolgt durch die Detektionseinrichtung 26, im Bereich eines Speichers 22 für eine Mehrzahl von auf Transportbügeln 15 hängenden Arbeitskitteln 11. Dieser Speicher 22 befindet sich in einer anderen Ebene, im gezeigten Ausführungsbeispiel einer höheren Ebene, insbesondere einer Etage über der Ebene bzw. Etage, an der mindestens eine Person 20 Arbeitskittel 11 auf den Transportbügel 15 aufhängt und/oder von mindestens einer Bedienungsperson 25 die Fremdkörper 37 nach ihrer Detektion aus den geröntgten textilen Gegenständen wie zum Beispiel Arbeitskitteln 11 entfernt werden.

Von der Detektionseinrichtung 26, die über mindestens eine Strahlenquelle 27, erfindungsgemäß eine Röntgenstrahlungsquelle, und wenigstens einen Detektor 28, insbesondere einen Zeilendetektor, verfügt, wird jeweils ein einzelner Arbeitskittel 11 bestrahlt bzw. durchleuchtet, vorzugsweise geröntgt, und dabei eventuelle Fremdkörper 37 sichtbar gemacht und ein Abbild des Arbeitskittels 11 mit eventuell darin vorhandenen Fremdkörpern 37 vom Detektor 28 erzeugt und/oder aufgenommen. Dadurch liefert der Detektor 28 Bilddaten vom jeweiligen inspizierten Arbeitskittel 11. Diese Bilddaten werden bevorzugt zugeordnet zum dazugehörenden Arbeitskittel 11 in einer Bilddatei oder als Bild abgespeichert und vorzugsweise mindestens einer Person 25 an der Fremdkörperentnahmestrecke 24 visuell angezeigt, insbesondere als Bild und/oder Röntgenbild.

Bei den in den Fig. 1 und 4 gezeigten Vorrichtungen sind die Detektionseinrichtungen 26 in einer Kammer 29 angeordnet. Diese Kammer dient zur Abschirmung der von der Strahlenquelle 27 erzeugten Röntgenstrahlen oder gleichwirkender elektromagnetischer Strahlen. Auch das trägt dazu bei, dass Personen, insbesondere Personen 20 und 25, die am dichtesten an der Detektionseinrichtung 26 mit der Strahlenquelle 27 arbeiten, keiner nennenswerten Strahlenbelastung ausgesetzt sind, aber mindestens die Strahlenbelastung unter den festgelegten Grenzwerten liegt. Wenn die Detektionseinrichtung 26 mit der Strahlenquelle 27 in der zur Abschirmung dienenden Kammer 29 angeordnet ist, brauchen der Speicher 22 bzw. seine Speicherstrecke, insbesondere aber die Detektionseinrichtung 26, nicht in einer anderen Ebene oder Etage angeordnet zu sein. Es können sich dann die Detektionseinrichtung 26 mit der Strahlenquelle 27 in der gleichen Etage befinden, in der Personen tätig sind, insbesondere die am nächsten an der Strahlenquelle tätigen Personen 20 und 25.

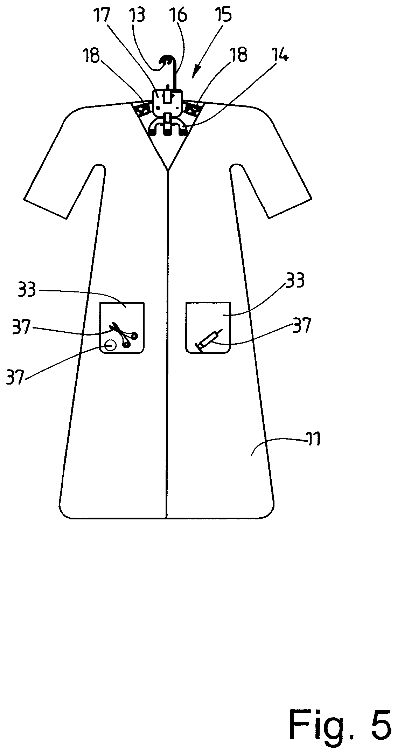

Die Fig. 5 zeigt schematisch ein von der Detektionseinrichtung 26 erzeugtes und aufgenommenes Bild, insbesondere einen Röntgenscan. Dieser zeigt die Umrisse des Arbeitskittels 11 samt seiner Taschen 33. Infolge des Röntgens des Arbeitskittels 11 mit elektromagnetischer Strahlung von der Detektionseinrichtung 26 werden auch Fremdkörper 37 in dem Arbeitskittel 11 sichtbar gemacht. Beispielhaft sind in der Fig. 4 als Fremdkörper 37 eine Münze, eine Schere und eine Spritze in den Taschen 33 dargestellt. Genauso können von der Detektionseinrichtung 26 andere Fremdkörper ermittelt werden, beispielsweise Schreibgeräte, auch solche, die sich nicht in den Taschen 33 des Arbeitskittels 11 befinden, sondern beispielsweise im Kragenbereich angesteckt sind. Auch können gegebenenfalls Namensschilder oder dergleichen als Fremdkörper 37 von der Detektionseinrichtung 26 erkannt und als Bild aufgenommen werden.

Die von der Detektionseinrichtung 26 auf das Vorhandensein von Fremdkörpern 37 inspizierten Arbeitskittel 11 gelangen nach dem Verlassen des Speichers 22 in den Bereich der Fremdkörperentnahmestrecke 24. Arbeitskittel 11, bei denen keine Fremdkörper 37 festgestellt wurden, werden kontinuierlich durch die Fremdkörperentnahmestrecke 24 hindurchtransportiert, zu beispielsweise einer Sortiereinrichtung oder einer Wäschebehandlungseinrichtung. Arbeitskittel 11 mit mindestens einem Fremdkörper 37 werden im Bereich der Fremdkörperentnahmestrecke 24 nahe der Person 25 gestoppt, so dass die kurzfristig stehen bleiben, damit die Person 25 sämtliche Fremdkörper 37 vom Arbeitskittel 11 oder aus demselben entfernen kann. Die Person 25 an der Fremdkörperentnahmestrecke 24 wird bei der Trennung der Fremdkörper 37 vom Arbeitskittel 11 unterstützt durch das von der Detektionseinrichtung 26 ermittelte Bild (Fig. 5), das zu diesem Zweck aus der Bilddatenbank abgerufen und auf dem Monitor 34 zugeordnet zum sich momentan vor der Person 25 befindenden Arbeitskittel 11 angezeigt wird. Auf dem Monitor 34 wird stets das Bild angezeigt, das zum momentan in der Nähe der Person 25 gestoppten Arbeitskittel 11 gehört. Anhand des Bildes auf dem Monitor 34 kann die Person 25 erkennen, wie viele Fremdkörper 37 sich am bzw. im Arbeitskittel 11 befinden, wo sich die Fremdkörper 37 befinden, beispielsweise in welcher Tasche 33 und/oder um welche Art von Fremdkörper 37 es sich handelt. Wird zum Beispiel als Fremdkörper 37 eine Spritze detektiert, weiß die Person 25, dass sie beim Entnehmen derselben aus der Tasche des Arbeitskittels 11 vorsichtig sein muss, um sich nicht zu verletzen.

Es ist denkbar, dass zusätzlich auf dem Monitor 34 der Person 25 angezeigt wird, wie viele Fremdkörper 37 im sich momentan vor ihr befindlichen Arbeitskittel 11 vorhanden sind. Die Person 25 weiß dann, wie viele Fremdkörper 37 sie dem jeweiligen Arbeitskittel 11 entnehmen muss. Gegebenenfalls kann es vorgesehen sein, dass die Person 25 die Entnahme jedes Fremdkörpers 37 auf dem Monitor 34 oder einer diesem zugeordneten Tastatur quittieren muss. Wenn zum Beispiel im Arbeitskittel 11 (wie bei der Fig. 5) drei Fremdkörper 37 vorhanden sind, wird der Arbeitskittel 11 erst weitertransportiert, wenn die Person 25 drei Mal das Entnehmen jeweils eines Fremdkörpers 37 aus den Taschen 33 des Arbeitskittels 11 quittiert hat.

Es sind alternative Ausgestaltungsmöglichkeiten der Erfindung denkbar, bei der mehrere Arbeitskittel 11 gleichzeitig inspiziert werden. Dann verfügt jede Detektionseinrichtung 26 mindestens über so viele Detektoren 28 wie Arbeitskittel 11 gleichzeitig detektiert werden. Es kann ausreichen, wenn auch bei mehreren gleichzeitig zu inspizierenden Arbeitskitteln 11 die Detektionseinrichtung 26 nur eine Strahlenquelle 27 aufweist. Gleichwohl ist es denkbar, dass bei mehreren gleichzeitig dem Röntgenscan unterzogenen Arbeitskitteln 11 die Detektionseinrichtung 26 mehrere Röntgenquellen oder sonstige Strahlenquellen 27 aufweist.

Zuvor wurde die Erfindung im Zusammenhang mit Arbeitskitteln 11 beschrieben. Darauf ist die Erfindung aber nicht beschränkt. Sie eignet sich zum Inspizieren jeglicher Art textiler Gegenstände auf Fremdkörpern 37, insbesondere jeglicher Bekleidungsstücke (Formteile) und auch Flachwäsche (Tischwäsche, Bettwäsche oder dergleichen).

Ein alternatives Ausführungsbeispiel der Erfindung, das vorzugsweise das zuvor beschriebene Ausführungsbeispiel ergänzt und/oder weiterbildet, bezieht sich auf textile Gegenstände, die mindestens einen Datenträger aufweisen. Bei den Datenträgern kann es sich um solche handeln, die optisch, optoelektronisch oder rein elektromagnetisch berührungslos auslesbar sind. Solche Datenträger können Aufkleber, Barcodes, QR-Codes und/oder Datenspeicher, beispielsweise RFID-Chips oder NFC-Chips, sein.

Vor der Inspektion der textilen Gegenstände wird einzeln der Datenträger jedes textilen Gegenstands, oder zumindest solcher textilen Gegenstände, die Datenträger aufweisen, berührungslos ausgelesen.

Anhand der vom betreffenden Wäschestück stammenden Daten können nähere Informationen über den textilen Gegenstand und/oder das Wäschestück gewonnen werden, insbesondere solche, die für die nachfolgende Inspektion des textilen Gegenstands hinsichtlich eventueller Fremdkörper von Interesse sein könnten. Bevorzugt wird aus den ermittelten bzw. aufgenommenen Daten des jeweiligen textilen Gegenstands ermittelt, um welche Art eines textilen Gegenstands es sich handelt, beispielsweise einen Arbeitskittel 11.

Mit den gewonnenen Erkenntnissen findet vor der eigentlichen Inspektion der textilen Gegenstände eine Kategorisierung statt, beispielsweise danach, ob und welche metallischen Bestandteile die zu inspizierenden textilen Gegenstände typischerweise aufweisen, beispielsweise Knöpfe oder Reißverschlüsse, und nach solchen textilen Gegenständen, die aufgrund ihrer Art üblicherweise keine Knöpfe, Reisverschlüsse oder andere Bestandteile aufweisen.

Die vor der Inspektion der textilen Gegenstände erfolgende Ermittlung aller oder einiger auf ihren Datenträgern abgespeicherten Daten führt zu einer zuverlässigen, insbesondere fehlerfreien, Inspektion von Fremdkörpern durch elektromagnetische Strahlen. Wenn zum Beispiel aus den Daten eines textilen Gegenstands sich ergibt, dass es sich um einen Arbeitskittel 11 mit metallischen oder auch nicht metallischen Knöpfen handelt, werden diese zwar auch von den elektromagnetischen Strahlen bei der Inspektion detektiert; aufgrund der Anordnung, Lage und/oder Form der Knöpfe des Arbeitskittels 11, können solche Knöpfe von anderen Fremdkörpern, die zusammen mit den Knöpfen bei der Inspektion durch elektromagnetische Strahlen detektiert worden sind, unterschieden werden. Die Knöpfe werden dann nicht als Fremdkörper detektiert oder bleiben bei der Auswertung der Inspektion unberücksichtigt. Wird hingegen beim Auslesen der Daten eines textilen Gegenstands festgestellt, dass es sich hierbei um ein T-Shirt handelt, das keinerlei zusätzliche Bestandteile aufweist, stellt jeder bei der nachfolgenden Inspektion mit elektromagnetischen Strahlen detektierte Gegenstand einen Fremdkörper dar.

Bei dem zuvor beschriebenen Ausführungsbeispiel, dass das eingangs beschriebene erste Ausführungsbeispiel vorzugsweise ergänzt, ist eine zumindest nahezu fehlerfreie Detektion von Fremdkörpern durch Beaufschlagung des zu prüfenden textilen Gegenstands mit elektromagnetischen Strahlen möglich, indem aufgrund des vorher ausgelesenen Datenträgers des jeweiligen textilen Gegenstands die Art desselben bekannt ist und somit der textile Gegenstand auf diese Weise identifiziert wird. Bei der Auswertung des Ergebnisses der Inspektion durch elektromagnetische Strahlen werden metallische und vorzugsweise auch nichtmetallische Gegenstände, beispielsweise Knöpfe und Reißverschlüsse, die der jeweils zu inspizierende Gegenstand aufweist, nicht als Fremdkörper detektiert und/oder dargestellt.

Ein anderes Ausführungsbeispiel der Erfindung, das vorzugsweise im Zusammenhang mit den vorstehend beschriebenen Ausführungsbeispielen, aber auch hiervon unabhängig, realisierbar ist, zeichnet sich dadurch aus, dass der jeweilige, vorzugsweise jeder, textile Gegenstand zur Ermittlung der Fremdkörper in oder an demselben mit Röntgenstrahlen mit mehreren unterschiedlichen Spektren inspiziert wird. Vorzugsweise erfolgt bei Röntgenstrahlen, jedes unterschiedlichen Spektrums, eine Ermittlung der Fremdkörper, indem mit jedem der unterschiedlichen Spektren vom gleichen textilen Gegenstand eine Aufnahme erzeugt wird, und anschließend die Aufnahmen vorzugweise überlagert und/oder verrechnet werden und daraus ein einziges, gemeinsames Bild erzeugt wird.

Bevorzugt ist es vorgesehen, den jeweiligen textilen Gegenstand mittels nur einer Röntgenstrahlung oder sonstiger elektromagnetischer Strahlung mit nur einem einzigen Spektrum bzw. einem bestimmten konstanten Spektrum zu inspizieren. Es reicht dann eine einzige Strahlenquelle, die nur ein bestimmtes Spektrum bzw. Röntgenspektrum, erzeugt, um die textilen Gegenstände zu inspizieren. Der jeweilige textile Gegenstand wird dann einer Röntgenstrahlung gleichen Spektrums ausgesetzt. Diese Röntgenstrahlung wird dann aber nicht direkt zu mindestens einem Detektor geleitet. Vielmehr ist dann dem mindestens einen Detektor ein Mittel vor- oder zugeordnet, dass das Spektrum der Röntgenstrahlung mindestens teilweise verändert. Dieses Mittel dämpft wenigstens einen Teil des Spektrums der von der Strahlenquelle stammenden Röntgenstrahlung. Dadurch wird die spektrale Zusammensetzung des vorzugsweise einzigen von der Strahlungsquelle erzeugten Spektrums der elektromagnetischen Strahlung geändert. Dadurch können hinter dem Mittel Röntgenstrahlen verschiedener Spektren und/oder verschiedener Spektralzusammensetzungen entstehen. Das Mittel zur Veränderung eines Teils des von der Strahlungsquelle stammenden einheitlichen Spektrums ist bevorzugt als ein Filter ausgebildet, der geeignet ist, die Röntgenstrahlung oder eine andere elektromagnetische Strahlung hinsichtlich ihres Spektrums zu verändern.

Die vom Filter durchgelassenen Röntgenstrahlen unterschiedlicher Spektren und/oder verschiedener spektraler Zusammensetzungen werden bevorzugt von einem einzigen Detektor erfasst, der getrennte Detektorbereiche aufweist, und zwar für jedes der unterschiedlichen Spektren einen eigenen Detektorbereich aufweist. Alternativ ist es aber auch denkbar, dass in Strahlungsrichtung hinter dem mindestens einen Filter mehrere getrennte Detektoren vorgesehen sind. Dabei ist für jedes Spektrum und/oder jeder spektralen Zusammensetzung dann ein eigener Detektor vorgesehen.

Das Verfahren nach dem zuvor beschriebenen Ausführungsbeispiel wird anhand eines Beispiels mit Röntgenstrahlen, deren Spektrum gedämpft worden ist, nachfolgend erläutert:
Die vorzugsweise einzige Strahlenquelle, insbesondere eine einzige Röntgenquelle, erzeugt Röntgenstrahlung eines Spektrums. Diese Röntgenstrahlung trifft nach dem Durchtritt durch den textilen Gegenstand oder einer Reflexion auf den mindestens einen Detektor, der vorzugsweise der der Röntgenquelle weggerichteten bzw. abgewandten Seite des zu inspizierenden textilen Gegenstands zugeordnet ist. Vor dem mindestens einen Detektor wird das Röntgenspektrum mindestens teilweise verändert. Dadurch entstehen aus der den jeweiligen textilen Gegenstand durchstrahlten oder von ihr reflektierten, vorzugsweise einheitlichen, Strahlung zwei verschiedene Röntgenspektren mit jeweils unterschiedlicher spektraler Zusammensetzung.

Die beiden unterschiedlichen Röntgenspektren werden gebildet durch mindestens einen vor dem oder an dem jeweiligen Detektor vorgesehenen Filter. Vom Filter gelangen dann Röntgenstrahlen zweier unterschiedlicher Spektren und/oder unterschiedlicher spektraler Zusammensetzungen zum Detektor, der bei zwei voneinander abweichenden Spektren als solcher mit zwei Detektorbereichen ausgebildet ist. Dieser Detektor wertet gleichzeitig, insbesondere simultan, die unterschiedlichen spektralen Zusammensetzungen aus, so dass zwei verschiedene Aufnahmen und/oder Bilddatensätze vom jeweils zu inspizierenden textilen Gegenstand entstehen. Diese werden dann elektronisch verrechnet und/oder überlagert zur Bildung eines einzelnen für eine Person wahrnehmbaren Bildes vom geröntgten textilen Gegenstand. Auf diesem Bild sind sowohl Fremdkörper, die vom einen Spektrum abgebildet worden sind als auch Fremdkörper, die vom zweiten Spektrum abgebildet sind, gleichzeitig enthalten. Die Person kann dann anhand eines Röntgenbilds vom inspizierten textilen Gegenstand alle Fremdkörper in und an demselben erkennen, und zwar Fremdkörper unterschiedlicher Materialien, vor allem Materialien, die kein Textil sind. Bei den Materialien, die kein Textil sind, muss es sich nicht zwingend um metallische Materialien handeln, es kann sich auch um metallische Materialien einerseits und nichtmetallische Materialien andererseits handeln, also Fremdkörper wie beispielsweise Kunststoffknöpfe, Taschentücher, insbesondere Papiertaschentücher, Kunststoffreißverschlüsse oder dergleichen.

Bei zum Beispiel Fremdkörpern aus zum einen Teil Kunststoff und zum anderen Teil Metall können aufgrund verschiedener Spektren Dichteinformationen von zum Beispiel Kunststoffmaterialien und metallischen Materialien erhalten werden. Durch Zusammensetzen und/oder Verrechnen der von unterschiedlichen Spektren erzeugten Aufnahmen, Bildern oder Bilddaten wird dann für den Betrachter ein einziges Bild erzeugt, das dem Betrachter Zusatzinformationen über das Material oder die Materialien des jeweiligen Fremdkörpers zur Verfügung stellt. So lassen sich beispielsweise Spritzen mit einem Kunststoff- bzw. Glaskörper und einer metallischen Kanüle für den Betrachter vollständig als solche erkennen.

## Patentansprüche

1. Verfahren zum Inspizieren textiler Gegenstände, insbesondere benutzter textiler Gegenstände, auf das Vorhandensein mindestens eines Fremdkörpers (37) in bzw. an den textilen Gegenständen mittels Röntgenstrahlen, **dadurch gekennzeichnet, dass** die Inspektion des jeweiligen textilen Gegenstands mit unterschiedlichen Spektren unterschiedlicher Energie von Röntgenstrahlen erfolgt, indem vom gleichen textilen Gegenstand mindestens eine Aufnahme zu allen Spektren der Röntgenstrahlen erzeugt wird und die Ermittlung von Fremdkörpern (37) im oder am jeweiligen textilen Gegenstand durch Auswertung sämtlicher mit verschiedenen Spektren vom zu inspizierenden textilen Gegenstand gemachten Aufnahmen erfolgt, wodurch Fremdkörper unterschiedlicher Materialien, insbesondere metallischer und nichtmetallischer Materialien, durch Dichteinformationen aufgrund der verschiedenen Spektren ermittelt werden.

2. Verfahren nach Anspruch 1, **dadurch gekennzeichnet, dass** die unterschiedlichen Spektren der Röntgenstrahlen gleichzeitig erzeugt werden und/oder die Röntgenstrahlen unterschiedlicher Spektren durch mindestens einen Detektor mit mehreren Detektorbereichen, insbesondere einem Detektorbereich für jedes Spektrum, aufgenommen werden, wobei vorzugsweise die mehreren Detektionsbereiche von unterschiedlichen Filtern oder unterschiedlichen Filterbereichen eines Filters zur Veränderung des Spektrums der Röntgenstrahlen gebildet werden.

3. Verfahren nach Anspruch 1 oder 2, **dadurch gekennzeichnet, dass** vom Detektor mit mehreren unterschiedlichen Detektionsbereichen jedes der unterschiedlichen Spektren der Röntgenstrahlen von einem eigenen Detektionsbereich aufgenommen wird und/oder bei mehreren Detektoren jedes der unterschiedlichen Spektren vom eigenen Detektor aufgenommen wird.

4. Verfahren nach einem der vorhergehenden Ansprüche, **dadurch gekennzeichnet, dass** die Ermittlung der Fremdkörper (37) mit Röntgenstrahlen an einer von Personen (20, 25) solchermaßen beabstandeten Stelle vorgenommen wird, dass eine Strahlenbelastung der Person (20, 25) unter einem maximal zulässigen Grenzwert liegt, mindestens die von den Röntgenstrahlen sichtbar gemachten Fremdkörper (37) durch wenigstens eine bildgebende Einrichtung aufgenommen werden und die dabei erhaltenen Daten für jeden einzelnen textilen Gegenstand, bei dem mindestens ein Fremdkörper (37) ermittelt worden ist, in einem Speicher abgespeichert werden oder die von der wenigstens einen bildgebenden Einrichtung aufgenommenen Daten als Bild des textilen Gegenstands mit dem mindestens einen durch die Röntgenstrahlen sichtbar gemachten Fremdkörper (37) wenigstens einer Person (25) visuell dargestellt werden.

5. Verfahren nach Anspruch 4, **dadurch gekennzeichnet, dass** die textilen Gegenstände im mindestens teilweise hängenden Zustand, vorzugsweise an einer Transporteinrichtung (10) hängenden und/oder auf einem Transportbügel (15) hängenden Zustand, der die Fremdkörper (37) detektierenden oder sichtbarmachenden Röntgenstrahlung ausgesetzt werden.

6. Verfahren nach Anspruch 4 oder 5, **dadurch gekennzeichnet, dass** die Detektion der Fremdkörper (37) am einzelnen textilen Gegenstand vorgenommen wird, vorzugsweise während des kontinuierlichen Weitertransports desselben.

7. Verfahren nach einem der vorhergehenden Ansprüche, **dadurch gekennzeichnet, dass** die Detektion der Fremdkörper (37) im Bereich einer Speicherstrecke und/oder eines Speichers (22) der textilen Gegenstände erfolgt und/oder die Detektion der Fremdkörper (37) in einer Etage erfolgt, die sich über einer Etage befindet, in der die Personen (20, 25) die textilen Gegenstände handhaben bzw. bearbeiten.

8. Verfahren nach einem der vorhergehenden Ansprüche, **dadurch gekennzeichnet, dass** nach dem Anhängen des jeweiligen textilen Gegenstands an die Transporteinrichtung (10), vorzugsweise nach dem Aufhängen des jeweiligen textilen Gegenstands auf den Transportbügel (15) oder dem Anhängen des textilen Gegenstands an den Transportbügel (15), die Detektion bzw. Inspektion des betreffenden textilen Gegenstands in Bezug auf Fremdkörper (37) vorgenommen wird.

9. Verfahren nach einem der vorhergehenden Ansprüche, **dadurch gekennzeichnet, dass** die textilen Gegenstände, vorzugsweise nur jeweils ein einzelner textiler Gegenstand oder nur wenige textile Gegenstände, in einer Kammer (29), insbesondere einer mindestens größtenteils geschlossenen Kammer (29) und/oder einer gegen den Austritt wenigstens eines Großteils der Röntgenstrahlen abgeschirmten Kammer (29), mit den Röntgenstrahlen beaufschlagt werden.

10. Verfahren nach einem der vorhergehenden Ansprüche, **dadurch gekennzeichnet, dass** die Länge der textilen Gegenstände für die Inspektion mit Röntgenstrahlen reduziert wird, vorzugsweise in der Kammer (29) reduziert wird, wobei insbesondere mindestens in der Kammer (29) ein Unterteil des jeweiligen textilen Gegenstands sich auf einem Boden (36) der Kammer (29) oder einer auf bzw. über dem Boden (36) angeordneten Führungsbahn (35) oder einem Gurtförderer abstützt.

11. Verfahren nach einem der vorhergehenden Ansprüche, **dadurch gekennzeichnet, dass** vor der Inspektion der textilen Gegenstände eine Identifikation und/oder Klassifikation derselben vorgenommen wird, insbesondere vor der Inspektion der textilen Gegenstände mit Röntgenstrahlen Datenträger der textilen Gegenstände ausgelesen werden, und daraus die Art des jeweiligen textilen Gegenstands ermittelt oder abgeleitet wird und/oder anhand der ausgelesenen Daten oder der ermittelten Art des jeweiligen textilen Gegenstands eine individuelle Auswertung, Bewertung und/oder Identifizierung des jeweiligen textilen Gegenstands und/oder des Ergebnisses der Inspektion des jeweiligen textilen Gegenstands, vorzugsweise der bei der Inspektion detektierten Fremdkörper, erfolgt.

## Claims

1. A method for inspecting textile articles, in particular used textile articles, for the presence of at least one foreign body (37) in or on the textile articles by means of X-rays, **characterized in that** the inspection of the respective textile article is carried out with different spectra of different energy of X-rays by generating at least one image of all X-ray spectra from the same textile article and detecting foreign bodies (37) in or on the respective textile article by evaluating all images taken with different spectra from the textile item to be inspected, whereby foreign bodies of different materials, in particular metallic and non-metallic materials, are detected by density information based on the different spectra.

2. The method as claimed in claim 1, **characterized in that** the different spectra of X-rays are produced simultaneously and/or the X-rays of different spectra are recorded by at least one detector having a plurality of detector regions, in particular one detector region for each spectrum, wherein the plurality of detection regions are preferably formed by different filters or different filter regions of a filter for modifying the spectrum of the X-rays.

3. The method as claimed in claim 1 or 2, **characterized in that** each of the different spectra of the X-rays is recorded by its own detection region by the detector having a plurality of different detection regions, and/or in the case of a plurality of detectors, each of the different spectra is recorded by its own detector.

4. The method as claimed in one of the preceding claims, **characterized in that** the determination of the foreign bodies (37) is carried out with X-rays at a position separated from persons (20, 25) such that a radiation exposure of the person (20, 25) lies below a maximum allowed limit value, at least the foreign bodies (37) made visible by the X-rays are recorded by at least one imaging device and the data thereby obtained are stored in a memory for each individual textile article for which at least one foreign body (37) has been determined or the data recorded by the at least one imaging device are visually represented to at least one person (25) as an image of the textile article with the at least one foreign body (37) made visible by the X-rays.

5. The method as claimed in claim 4, **characterized in that** the textile articles are exposed to X-radiation, which detects the foreign bodies (37) or makes them visible, in the at least partially hanging state, preferably a state hanging from a transport device (10) and/or hanging on a transport hanger (15).

6. The method as claimed in claim 4 or 5, **characterized in that** the detection of the foreign bodies (37) is carried out on the individual textile article, preferably during continuous further transport thereof.

7. The method as claimed in one of the preceding claims, **characterized in that** detection of the foreign bodies (37) is carried out in the region of a storage section and/or a storage area (22) for the textile articles, and/or the detection of the foreign bodies (37) is carried out in a level which lies above a level in which the persons (20, 25) handle or process the textile articles.

8. The method as claimed in one of the preceding claims, **characterized in that** the detection or inspection of the relevant textile article in respect of foreign bodies (37) is carried out after the hanging of the respective textile article from the transport device (10), preferably after the hanging of the respective textile article on the transport hanger (15) or the hanging of the textile article from the transport hanger (15).

9. The method as claimed in one of the preceding claims, **characterized in that** the textile articles, preferably only in each case an individual textile article or only a few textile articles, are exposed to X-rays in a chamber (29), in particular an at least mostly closed chamber (29) and/or a chamber (29) shielded against the emergence of at least a majority of X-rays.

10. The method as claimed in one of the preceding claims, **characterized in that** the length of the textile articles is reduced for the inspection with X-rays, preferably reduced in the chamber (29), in particular with a lower part of the respective textile article being supported on a floor (36) of the chamber (29) or on a guide surface (35) arranged on or above the floor (36) or on a belt conveyor, at least in the chamber (29).

11. The method as claimed in one of the preceding claims, **characterized in that** an identification and/or classification of the textile articles is carried out before the inspection thereof, in particular that data carriers of the textile articles are read out before the inspection of the textile articles with X-rays, and the type of the respective textile article is determined or derived therefrom, and/or on the basis of the read data or the determined type of the respective textile item, an individual evaluation, assessment and/or identification of the respective textile item and/or of the result of the inspection of the respective textile item, preferably of the foreign bodies detected during the inspection, is carried out.

## Revendications

1. Procédé d'inspection d'articles textiles, en particulier d'articles textiles utilisés, pour détecter la présence d'au moins un corps étranger (37) dans ou sur les articles textiles au moyen de rayons X, **caractérisé en ce que** l'inspection de l'article textile respectif est effectuée avec différents spectres de différentes énergies de rayons X, **en ce qu'**au moins une prise de vue à tous les spectres des rayons X du même article textile est produite et la détermination de la présence de corps étrangers (37) dans ou sur l'article textile respectif s'effectue par l'évaluation de toutes les prises de vue réalisées avec différents spectres de l'article textile à inspecter, moyennant quoi les corps étrangers de matériaux différents, notamment des matériaux métalliques et non métalliques, sont déterminées par des informations de densité sur la base des différents spectres.

2. Procédure selon la revendication 1, **caractérisée en ce que** les différents spectres des rayons X sont générés simultanément et/ou les rayons X de différents spectres sont captés par au moins un détecteur doté de plusieurs zones de détection, en particulier une zone de détection pour chaque spectre, les plusieurs zones de détection étant de préférence formées par différents filtres ou de différentes zones de filtrage d'un filtre en vue de modifier le spectre des rayons X.

3. Procédé selon la revendication 1 ou 2, **caractérisé en ce que** le détecteur doté de plusieurs zones de détection différentes capture chacun des différents spectres des rayons X à partir d'une zone de détection distincte et/ou, dans le cas de plusieurs détecteurs, chacun des différents spectres est capté par son propre détecteur.

4. Procédé selon l'une des revendications précédentes, **caractérisé en ce que** la détermination des corps étrangers (37) par rayons X est effectuée à un endroit éloigné des personnes (20, 25) de telle sorte que l'exposition de la personne (20, 25) aux rayonnements est inférieure à la valeur limite maximale admissible, au moins les corps étrangers (37) rendus visibles par les rayons X sont capturés par au moins un dispositif d'imagerie et les données ainsi obtenues pour chaque article textile individuel pour lequel au moins un corps étranger (37) a été déterminé sont enregistrées dans une mémoire ou les données capturées par l'au moins un dispositif d'imagerie sont représentées visuellement à au moins une personne (25) sous la forme d'une image de l'article textile avec l'au moins un corps étranger (37) rendu visible par les rayons X.

5. Procédé selon la revendication 4, **caractérisé en ce que** les articles textiles sont exposés aux rayons X détectant ou rendant visible les corps étrangers (37) dans un état au moins partiellement suspendu, de préférence à un dispositif de transport (10) et/ou dans un état suspendu à un cintre de transport (15).

6. Procédé selon la revendication 4 ou 5, **caractérisé en ce que** la détection des corps étrangers (37) est effectuée sur l'article textile individuel, de préférence pendant la poursuite du transport continu de celui-ci.

7. Procédure selon l'une des revendications précédentes, **caractérisée en ce que** la détection des corps étrangers (37) s'effectue dans la zone d'un parcours de stockage et/ou d'un entrepôt (22) des articles textiles et/ou la détection des corps étrangers (37) s'effectue dans un étage qui se trouve au-dessus d'un étage auquel les personnes (20, 25) manipulent ou traitent les articles textiles.

8. Procédure selon l'une des revendications précédentes, **caractérisée en ce que** la détection ou l'inspection de l'article textile respectif en vue de la détection de corps étrangers (37) est effectuée après avoir suspendu l'article textile respectif au dispositif de transport (10), de préférence après avoir suspendu l'article textile respectif au cintre de transport (15) ou après avoir attaché l'article textile respectif au cintre de transport (15).

9. Procédure selon l'une des revendications précédentes, **caractérisée en ce que** les articles textiles, de préférence à chaque fois un seul article textile ou seulement un petit nombre d'articles textiles, sont exposés aux rayons X dans une chambre (29), en particulier une chambre (29) fermée au moins en grande partie et/ou une chambre (29) blindée contre la sortie d'au moins une grande partie des rayons X.

10. Procédé selon l'une des revendications précédentes, **caractérisé en ce que** la longueur des articles textiles est réduite pour l'inspection par rayons X, de préférence réduite dans la chambre (29), une partie inférieure de l'article textile respectif, notamment au moins dans la chambre (29), reposant sur un sol (36) de la chambre (29) ou sur une glissière de guidage (35) ou un convoyeur à bande situé sur ou au-dessus du sol (36).

11. Procédé selon l'une des revendications précédentes, **caractérisé en ce qu'**une identification et/ou une classification des articles textiles est effectuée avant l'inspection de ceux-ci, les supports de données des articles textiles sont notamment lus avant l'inspection des articles textiles par rayons X et la nature de l'article textile respectif est déterminée ou dérivées à partir de ceux-ci et/ou une analyse, une évaluation et/ou une identification individuelles de l'article textile respectif et/ou du résultat de l'inspection de l'article textile respectif étant effectuée à l'aide des données lues ou de la nature déterminée de l'article textile respectif, de préférence des corps étrangers détectés lors de l'inspection.
